# EUROPÄISCHE PATENTSCHRIFT

(11) **EP 3 995 102 B1**
(45) Veröffentlichungstag und Bekanntmachung des Hinweises auf die Patenterteilung: **28.01.2026**
(21) Anmeldenummer: 20206276.6
(22) Anmeldetag: 06.11.2020
(51) Int. Cl.: A61B 17/86, A61C 8/02, A61C 8/00, A61B 17/88

(54) **KNOCHENAUFBAU-SCHRAUBE SOWIE SYSTEM ZUR MODIFIKATION EINES MENSCHLICHEN ODER TIERISCHEN KNOCHENS**
BONE SCREW AND SYSTEM FOR MODIFICATION OF A HUMAN OR ANIMAL BONE
VIS DE RECONSTITUTION OSSEUSE AINSI QUE SYSTÈME DE MODIFICATION D'UN OS HUMAIN OU ANIMAL

(43) Veröffentlichungstag der Anmeldung: 11.05.2022
(73) Patentinhaber: Schlee, Markus, 91301 Forchheim (DE)
(72) Erfinder: Schlee, Markus, 91301 Forchheim (DE)
(74) Vertreter: Tergau & Walkenhorst Intellectual Property GmbH

(56) Entgegenhaltungen:
- DE-T2- 69 533 129
- DE-U1- 9 017 101
- US-A1- 2004 073 218
- US-A1- 2005 059 864
- US-A1- 2005 192 578
- US-A1- 2015 209 082

## Beschreibung

Die Erfindung betrifft eine zur Verwendung als Knochenaufbau-Schraube vorgesehene Schraube zur Verwendung bei der Modifikation eines menschlichen oder tierischen Knochens, insbesondere zum therapeutischen Einsatz im Bereich der plastischen oder dentalen Chirurgie. Sie betrifft weiter eine an diese angepasste Gewindemutter sowie ein System zur Modifikation eines menschlichen oder tierischen Knochens.

Bekannten Schrauben zur Verwendung bei der Modifikation eines Knochens sind aus DE69533129 T2, US2005/059864 A1, US2005/192578 A1, US2004/073218 A1 und DE9017101 U1 bekannt.

Insbesondere im Bereich der plastischen oder dentalen Chirurgie kann das Bedürfnis einer gezielten Modifikation des vorhandenen Knochens bestehen. Dabei können insbesondere additive Modifikationen, bei denen der vorhandenen Knochenmasse weitere Knochenmasse hinzugefügt wird, wünschenswert sein.

Derartige additive Modifikationen im Sinne eines Knochenaufbaus können insbesondere im Bereich der Traumatologie, plastischen Chirurgie oder Implantologie, beispielsweise der dentalen Implantologie, notwendig oder wünschenswert sein, um eine für eine sichere Verankerung eines Implantats in ausreichendem Knochenvolumen mit ausreichend hoher Primärstabilität ausreichende Knochensubstanz bereitstellen zu können. Ein Knochenaufbau wird nämlich in der Regel immer dann notwendig, wenn die Menge an vorhandenem Knochen für die Implantation nicht ausreicht oder die Kieferknochenstruktur nicht stimmt, um das Zahnimplantat fest im Kiefer zu verankern. Grund hierfür kann beispielsweise Knochenschwund oder Knochenabbau sein, der in Folge von Infektionen, Zahnlosigkeit oder Zahnerkrankungen entstanden ist und ohne eine adäquate Behandlung weiter fortschreitet. Dann ist auch das übrige Gebiss von schweren Folgen für die Zahngesundheit und weiterem Zahnverlust bedroht. Im Allgemeinen wird in der Chirurgie fehlender Knochen aufgebaut oder überschüssiger Knochen entfernt; falls dies erforderlich ist oder gewünscht wird. Knochenaufbau kann beispielsweise nach Unfall, Tumorentfernung, Zahnverlust oder in der kosmetischen, bzw. plastischen Chirurgie erforderlich sein.

Falls ein Knochenaufbau erforderlich werden sollte, kann das Knochenvolumen in der Art einer Transplantation autogenen Knochens vermehrt werden, indem andernorts Knochen entnommen und an der Empfängerstelle wieder eingesetzt wird. Nachteilig ist bei einer solchen Methode, dass zwei Operationen am Patienten durchgeführt werden müssen und sich dadurch das Komplikationsrisiko erhöht. Alternativ kann das Knochenvolumen aber auch durch Zuführung von so genanntem Knochenersatzmaterial vermehrt werden. Dabei werden üblicherweise Implantate aus Knochenersatzmaterialien (menschlichem oder tierischem Spenderknochen oder synthetischen Materialien, insbesondere alloplastischem Material) hergestellt und in den aufzubauenden Bereich eingebracht. Allogener Spenderknochen birgt jedoch ein immunologisches und/oder ein Infektionsrisiko in sich. Die verfügbare Menge an Spendermaterial ist zudem aus ethischen und juristischen Gründen limitiert. Beim alternativ möglichen Interspeziestransfer von Biomaterialien (Materialien tierischen Ursprunges, xenogenes Knochenersatzmaterial) kann es immunologische Probleme und Limitationen geben, die aus strukturellen oder anatomischen Unterschieden entstehen. In der Regel wird implantiertes xenogenes Knochenersatzmaterial zumindest für lange Zeit im Knochenregenerat oder sogar nur bindegewebig eingeschieden Die biologische Potenz zur Knochnregeneration solcher Materialien ist limitiert.

Im Vergleich zum autogenen Knochentransplantat entstehen somit im Ergebnis einer Implantation synthetischer Knochenersatzmaterialien Substitute, die ausnahmslos und vor allem bei den keramischen Implantaten als unvollständige Restitution zu werten sind. Ein ideales Knochensubstitut gibt es derzeit nicht.

In jedem Fall und unabhängig davon, welches dieser Knochenersatzmaterialien genau gewählt wird, ist es wünschenswert, das partikuläre Knochenersatzmaterial oder den partikulären Knochen vor Druck des umliegenden Gewebes, der Zunge, Wange, Schleimhaut, etc. zu schützen. Um den Knochen besonders wirksam zu regenerieren, braucht man Raum, Ruhe und ein Gerüst. Kochenblöcke (d. h. massive Stücke) können aus dem Kieferwinkel, Beckenkamm oder vom anderen Menschen (allogen) gewonnen werden. Diese schaffen den Raum und Ruhe selbst, wenn man sie anschraubt. Partikuläres Material hingegen ist nicht volumenstabil, wird aber deutlich leichter und schneller durchbaut.

Hinzu kann gerade bei signifikantem Knochen- oder Substanzverlust im Bereich des Kieferkamms das weitere Problem treten, dass mit vergleichsweise dünnwandigen noch verbleibenden Reststrukturen intakten Knochenmaterials gearbeitet werden muss, die eine nur vergleichsweise geringe mechanische Stabilität und Belastbarkeit aufweisen und somit eine mechanische Abstützung bei der Durchführung von Augmentationsmassnahmen in nur geringem Umfang bieten.

Der Erfindung liegt daher die Aufgabe zugrunde, eine für die Durchführung von Knochenaufbau-Massnahmen auch in schwierigem Umfeld besonders geeignete Schraube zur Verwendung bei der Modifikation eines menschlichen oder tierischen Knochens, insbesondere zum therapeutischen Einsatz im Bereich der plastischen oder dentalen Chirurgie anzugeben, mit der auf besonders einfache und insbesondere auch besonders gut verträgliche Weise zuverlässig ein Knochenaufbau gerade auch bei der Verwendung partikulären Materials erreicht werden kann. Des Weiteren sollen eine zur Verwendung in Kombination mit der Knochenaufbau-Schraube besonders geeignete Gewindemutter sowie ein für die vorgesehenen Knochenaufbau-Massnahmen besonders geeignetes System angegeben werden. Bezüglich der Schraube wird diese Aufgabe erfindungsgemäß gelöst mit einem in einem Gewindebereich mit einem Gewinde versehenen Schraubenschaft, an dessen erstem Schaftende ein im Wesentlichen konvex geformter Schraubenkopf angeordnet ist, und dessen Gewindebereich mindestens zwei Gewindesegmente umfasst, wobei in einem ersten, distalen, im Bereich des dem Schraubenkopf gegenüberliegenden Endes des Schraubenschafts positionierten Gewindesegment das Gewinde als metrische ISO-Gewindeschraube mit einem Außendurchmesser von weniger als dem Außendurchmesser, vorzugsweise von weniger als dem Kerndurchmesser, des Gewindes in einem zweiten, am Schraubenschaft zwischen dem Schraubenkopf und dem ersten Gewindesegment angeordneten**,** als selbstschneidendes Gewinde Gewindesegment ausgeführt ist.

Die Erfindung geht von der Überlegung aus, dass für eine besonders gut verträgliche und auch zuverlässige Modifikation von Knochenvolumen auf die natürlichen Mechanismen der Knochenbildung und des Knochenwachstums zurückgegriffen werden sollte. Die natürliche Bildung von Knochen, oder die Remodellation oder die Heilung einer Knochenverletzung, erfolgt durch Osteoblasten. Diese knochenbildenden Zellen und deren Vorläufer finden sich in der Knochenhaut (Periost), um die Blutgefäße (Perizyten), in der inneren Knochenhaut (Endost) und im Knochen selbst. Bei expandiertem Knochengewebe, beispielsweise bei Spaltbildung infolge von Knochenbrüchen, wandern aus den Gefäßwänden des expandierten Gewebes Perizyten aus, die sich zu Osteoprogenitorzellen und später zu Osteoblasten entwickeln. Gefäßanastomosen aus dem Knochen (Volkmannsche Gefäße) sind mit Gefäßen im Stratum fibrosum des Periosts verbunden. Die Osteoprogenitorzellen oder Osteoblasten im Kambium des Periosts sind ebenso an der Synthese neuen Knochens beteiligt. Daher führen Deformationen des Periosts, speziell die graduelle Distraktion von der Knochenoberfläche, zur Knochenneubildung (Knochenauflagerung) an einer bestehenden Knochenoberfläche.

Unter gezielter Nutzung dieser Erkenntnisse ist nunmehr vorgesehen, zum Zwecke des Knochenaufbaus gezielt einen Freiraum oberhalb des Knochens und unterhalb des Periosts oder der Knochenhaut zu schaffen, in den die Perzyten einwandern und nachfolgend eine Knochenneubildung bewirken können. Um den Freiraum zu schaffen, wurden in der Vergangenheit Metallplatten oder Gitter eingebracht. Das Weichgewebe wurde durch das Weichgewebe perforierende Metallteile mechanisch vom Knochen abgehoben. Dieses Verfahren birgt durch die Perforation ein Infektionsrisiko, und die gewünschte Form des Knochens lässt sich nur ungenügend regenerieren. Demgegenüber ist nunmehr zur Schaffung des genannten Freiraums erfindungsgemäß und in als eigenständig erfinderisch angesehener Ausgestaltung eine Schraube mit einem spezifisch auf diesen vorgesehenen Zweck ausgestalteten, vergleichsweise flächig ausgeführten Schraubenkopf vorgesehen, der für den Prozess des Knochenaufbaus zwischen Knochen und Periost eingeschoben und im Knochen inseriert oder verankert wird. Der Schraubenkopf hebt dabei das Periost relativ zum Knochen an und stützt es diesem gegenüber ab. Falls hingegen kein Periost vorhanden ist, wird ebenso oberhalb des Knochens ein Raum offengehalten, mit Partikeln (autolog, allogen, xenogen, synthetisch oder ein Gemisch aus diesen) gefüllt und mit oder ohne Membran abgedeckt und das Gewebe darüber vernäht. In beiden Fällen wird der besagte Freiraum gezielt gebildet, und der Knochenaufbau kann stattfinden.

In als eigenständig erfinderisch angesehener Ausgestaltung ist der Schraubenkopf im Hinblick auf diesen Einsatzzweck geeignet geformt. Dabei wird bevorzugt insbesondere den Kriterien Rechnung getragen, dass der Schraubenkopf bei montierter oder in den Knochen inserierter Schraube eine vergleichsweise große Auflagefläche für das Periost bilden sollte, so dass zu große punktuelle Belastungen des Periosts und damit einhergehend Risse, Perforationen oder Verletzungen im Periost vermieden oder zumindest minimiert werden. Im anderen Fall, in dem kein Periost im fraglichen Bereich vorhanden ist, sollte der Schraubenkopf durch seine Formgebung und Dimensionierung einen besonders geeigneten Raumbereich offen halten, in dem der Knochenaufbau stattfinden kann. Hierzu weist der Schraubenkopf, im Hinblick auf die bevorzugte Stärke oder den bevorzugten Durchmesser des Schraubenschaftes von beispielsweise 1,2 mm, eine entsprechend vergrößerte Fläche, bevorzugt einen Außendurchmesser von 3 bis 7 mm, besonders bevorzugt von etwa 5 mm, auf. In dieser besonders bevorzugten und als eigenständig erfinderisch angesehenen Ausführungsform weist der Schraubenkopf somit eine dem Periost zugewandte Auflagefläche von dem 20- bis 25-Fachen der Querschnittsfläche des Schraubenschaftes auf.

Des Weiteren ist die Konturierung des Schraubenkopfs vorteilhafterweise derart gewählt, dass auch formbedingt die Möglichkeit oder Wahrscheinlichkeit einer Verletzung oder Perforation des Periosts oder des darüber liegenden Hautlappens weitgehend minimiert ist. Hierzu ist der Schraubenkopf bevorzugt vergleichsweise flach und ohne scharfe Kanten in der Auflagefläche, in besonders bevorzugter Ausführung im Wesentlichen konvex, ausgeführt. Ziel der Formgestaltung ist dabei insbesondere, dass der Schraubenkopf das Periost zeltartig abstützt, wobei die eingetragenen Kräfte auf eine vergleichsweise große Fläche harmonisch verteilt werden.

Ergänzend dazu sollte auch der Schraubenschaft im Hinblick auf die genannten Auslegungsziele geeignet ausgeführt sein. Einerseits ist dabei dem Umstand Rechnung getragen, dass über den Schraubenschaft eine stabile und belastbare Verankerung der Schraube im Knochenmaterial erfolgen kann, so dass der Schraubenkopf in der Art eines Schirms über den Schraubenschaft getragen und somit als Auflagefläche für das Periost genutzt werden kann. Um dies zu ermöglichen, sollte der Schraubenschaft zumindest in einem Schaftsegment mit einem geeignet gewählten Außengewinde versehen sein, über das die sichere Verankerung im Knochen ermöglicht ist.

Um bei einer solchen Auslegung eine noch weiter verbesserte Nutzbarkeit im Einsatz in der dentalen Therapie zu ermöglichen, ist vorliegend als zusätzliche Komponente noch vorgesehen, dass der Schraubenschaft auch auf seinem eigentlich freien, dem Schraubenkopf gegenüberliegenden Ende mit einer als Trägerfläche z. B. für Periost nutzbaren Auflagefläche ausgerüstet werden kann. Eine solche Ausführung könnte insbesondere dann zum Tragen kommen, wenn die verbleibende Knochensubstanz vergleichsweise dünnwandig ist, so dass mit einem Durchstoßen des Schraubenschafts bei seiner Einbringung in den Knochen zu rechnen ist. Um die Schraube auch in einer solchen Situation einsetzbar zu machen, sollte an ihrem freien Ende eine Gewindemutter aufschraubbar sein, mit der im Bedarfsfall die weitere Auflagefläche bereitgestellt werden kann. Die vorliegend vorgesehene Schraube umfasst an ihrem Schraubenschaft somit zwei mit zueinander unterschiedlich ausgeführten Gewinden bestückte Segmente, von denen eines zur Verankerung im Knochen dient, und von denen das andere mit einem als Gewindeschraube ausgeführten Gewinde versehen ist, auf das ein dazu exakt passendes Innengewinde der Gewindemutter aufgeschraubt werden kann. Um dabei eine exakte Positionierung der Gewindemutter zu gewährleisten, sollten die Gewindeparameter, insbesondere die jeweiligen Durchmesser, geeignet gewählt sein, so dass der Übergang von dem zur Verankerung vorgesehenen zu dem für die Befestigung der Mutter vorgesehenen Segment eine geeignete Anschlagfläche für die Mutter bilden kann.

Vorteilhafte Ausgestaltungen der Erfindung sind Gegenstand der Unteransprüche.

Dieses Gewinde ist ähnlich zu den weithin in Dentalimplantaten verwendeten Gewinden als selbstschneidendes Gewinde ausgeführt. Eine solchermaßen ausgeführte Knochenaufbauschraube, umfassend den in zumindest einem Schaftsegment mit einem selbstschneidenden Außengewinde versehenen Schraubenschaft und den gemäß den vorstehenden Angaben ausgeführten Schraubenkopf, wird als eigenständig erfinderisch angesehen.

Bezüglich der Defintion der jeweiligen Gewindetypen wird auf die weitläufig akzeptierte Konvention zurückgegriffen, dass eine selbstschneidende Schraube ihr Innengewinde selbst schneidet, wenn sie in ein weicheres Material eingeführt wird, während eine Gewindeschraube in eine vorgebohrte Öffnung eingeführt wird und dann mit einer Mutter, deren Innengewinde exakt auf das Außengewinde der Schraube passt, angezogen wird.

Die Knochenaufbauschraube ist in besonders vorteilhafter Ausführung für eine erleichterte, dem erfolgreichen Knochenaufbau nachfolgende Wiederentnahme aus dem Knochen ausgeführt. Dazu ist die Schraube besonders bevorzugt aus einem geeigneten nicht-osseointegrierbaren Material wie beispielsweise medizinischem Stahl gefertigt. In alternativer oder zusätzlicher vorteilhafter Weiterbildung weist darüber hinaus der Schraubenschaft zusätzlich zum Gewindebereich einen gewindefreien, bevorzugt benachbart zum Schraubenkopf angeordneten Schaftbereich mit einem Schaftdurchmesser von mindestens 0,8 mm und höchstens 1,5 mm, bevorzugt von etwa 1,2 mm, auf. Durch diesen in der Art eines "frei liegenden Halses" ausgeführten Schaftbereich wird die mögliche Anhaftung des gebildeten Knochens an das Schraubensegment reduziert und die spätere Entnahme somit besondere begünstigt.

In weiterer vorteilhafter Ausgestaltung weist das Gewinde der Schraube im zweiten Gewindesegment einen Kerndurchmesser von mindestens 0,8 mm und höchstens 1,5 mm, bevorzugt von etwa 1,2 mm, auf. Durch eine solche Parameterwahl, insbesondere in Kombination mit dem oben genannten Parameterbereich für den gewindefreien Schaftbereich, wird ein insgesamt vergleichsweise dünn gehaltener Schraubenschaft bereitgestellt, der einerseits dem erwünschten Knochenaufbau vergleichsweise viel Raum bietet und ihn dadurch begünstigt, und der andererseits ein vergleichsweise leichtes Entfernen der Schraube aus dem Knochen nach dem erfolgten Knochenaufbau ermöglicht.

Diese Parameterwahl ist im übrigen ganz besonders günstig und bevorzugt in Kombination mit der genannten Parameterwahl, insbesondere eines Durchmessers von etwa 5mm, für den Schraubenkopf, denn in einer solchen Kombination geht ei vergleichsweise dünn gehaltener Schaft einher mit einem schirmartigen, vergleichsweise weit auskragenden und eine Trägerfläche für das Periost vom 20-bis 25-Fachen der Querschnittsfläche des Schafts bietenden Schraubenkopf.

Im ersten Gewindesegment ist das Gewinde als metrisches ISO-Gewinde, bevorzugt als M-Gewinde mit einem Außendurchmesser von mindestens 0,8 mm und höchstens 1,3 mm, bevorzugt von etwa 1 mm, ausgeführt. Bezüglich der insbesondere zur Verwendung mit einer Schraube der genannten Art vorgesehenen Gewindemutter wird die genannte Aufgabe gelöst mit einem im Wesentlichen tellerartig ausgeführt Grundkörper, der in einem Zentralbereich mit einer ein von seiner Parameterwahl an das Gewinde der Schraube angepasstes Innengewinde tragenden Gewindebohrung versehen ist. Eine solche Gewindemutter könnte im Bedarfsfall eine weitere Trägerfläche für das Periost oder aber auch bedarfsweise zur Abstützung an anderen geeigneten Stellen im zu therapierenden Bereich bieten.

Bei der Insertion der Knochenaufbau-Schraube muss im Allgemeinen mit einer sehr begrenzten räumlichen Flexibilität und äußerst beengten Platzverhältnissen gerechnet werden. Dies kann dazu führen, dass die Montage der Gewindemutter, also das Aufschrauben ihres Innesgewindes auf das erste Gewindesegment des Schraubenschafts, nur erschwert möglich sein wird. Um dem Rechnung zu tragen, ist die Gewindemutter in ganz besonders bevorzugter Ausgestaltung mit einer Montagehilfe versehen, die das Aufschrauben auf das erste Gewindesegment erleichtert. Dazu könnte an den Grundkörper des Gewindekörpers über eine Sollbruchstelle ein Montage-Hilfskörper angeformt sein, der nach dem Aufschrauben beispielsweise durch Abbrechen im Bereich der Sollbruchstelle auf besonders einfache Weise entfernt werden kann.

Alternativ oder zusätzlich ist der Grundkörper der Gewindemutter, bezogen auf seine Längsachse, in vorteilhafter Ausgestaltung mit einer nicht runden, vorzugsweise als Mehrkant, besonders bevorzugt als Vier- oder Sechskant, ausgeführten Außenkontur ausgestaltet. Damit ist ein herkömmliches Werkzeug, beispielsweise in der Art eines Maulschlüssels oder dergleichen, nutzbar, um die Anbringung zu erleichtern.

Bezüglich des Systems zur Modifikation eines menschlichen oder tierischen Knochens wird die genannte Aufgabe gelöst mit einer Schraube der vorgenannten Art, und mit einer hinsichtlich ihres Innengewindes an das Gewinde des ersten Gewindesegments der Schraube angepassten Gewindemutter der beschriebenen Art.

Die mit der Erfindung erzielten Vorteile bestehen insbesondere darin, dass durch die Ausgestaltung der Knochenaufbau-Schraube, insbesondere auch durch die Formgebung ihres Schraubenkopfs, auf vergleichsweise schonende und Verletzungen oder Perforationen des Periosts vermeidende Art und Weise eine Bereitstellung und künstliche Vorgabe eines Hohlraums zwischen Periost und Knochen ermöglicht ist, der das Knochenwachstum begünstigt. Des weiteren ist die Schraube aufgrund ihrer Ausgestaltung mit den zwei Gewindesegmenten in der Art eines in großer Anzahl und damit kostengünstig bereitstellbaren Standard-Bauteils für unterschiedliche Nutzungsarten geeignet, nämlich einerseits singulär, wobei lediglich über das selbstschneidende Gewinde eine Verankerung im Knochen erfolgt, oder auch in Kombination mit der Gewindemutter, bei der in entsprechenden Behandlungssituationen zusätzlich zum Schraubenkopf über die Gewindemutter noch eine weitere Trägerfläche für das Periost bereitgestellt werden kann.

Ein Ausführungsbeispiel der Erfindung wird anhand einer Zeichnung näher erläutert. Darin zeigen:
- FIG. 1: eine Knochenaufbau-Schraube,
- FIG. 2: eine zur Verwendung mit der Schraube nach FIG. 1 vorgesehene Gewindemutter,
- FIG. 3: eine Anzahl von jeweils aus einer Komnination einer Schraube gem. FIG. 1 und einer Gewindemutter nach FIG. 2 gebildeten Systemen zum Knochenaufbau im inserierten Zustand, und
- FIG. 4: die Gewindemutter nach FIG. 2 mit Montagehilfe.

Gleiche Teile sind in allen Figuren mit denselben Bezugszeichen versehen.

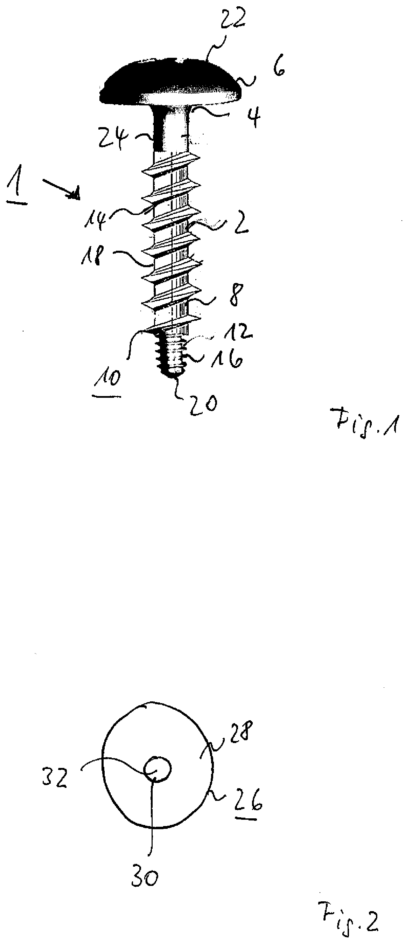

Die Schraube 1 gemäß FIG. 1 ist zur Verwendung bei der Modifikation eines menschlichen oder tierischen Knochens, insbesondere zum therapeutischen Einsatz im Bereich der plastischen oder dentalen Chirurgie, auf besonders schonende und für den Patienten verträgliche Weise vorgesehen. Sie umfasst einen Schraubenschaft 2, an dessen erstem Schaftende 4 ein Schraubenkopf 6 angeordnet ist. Der Schraubenschaft 2 ist in einem Gewindebereich 8 mit einem Gewinde 10 versehen. Der Gewindebereich 8 ist dabei segmentiert ausgeführt und umfasst im Ausführungsbeispiel zwei Gewindesegmente 12, 14, die sich in der Art ihres jeweiligen Gewindes 16, 18 voneinander unterscheiden. Im ersten, distalen, im Bereich des dem Schraubenkopf 6 gegenüberliegenden Endes 20 des Schraubenschafts 2 positionierten Gewindesegment 12 ist das Gewinde 10 als Gewindeschraube 16 ausgeführt, wohingegen es im zweiten, am Schraubenschaft 2 zwischen dem Schraubenkopf 6 und dem ersten Gewindesegment 12 angeordneten Gewindesegment 14 als selbstschneidendes Gewinde 18 ausgestaltet ist.

Der Schraubenkopf 6 ist im Hinblick auf die nachfolgende beschriebene Verwendung geeignet ausgeführt und konturiert, insbesondere um eine vergleichsweise große, flache und kanten- oder eckenfreie Auflagefläche 22 auszubilden. Dazu weist der Schraubenkopf 6, im Hinblick auf die bevorzugte Stärke oder den vorgesehenen Durchmesser des Schraubenschaftes 2 von im Ausführungsbeispiel 1,2 mm, einen entsprechend vergrößerten Außendurchmesser von etwa 5 mm auf. Des Weiteren ist der Schraubenkopf 6 vergleichsweise flach und im Wesentlichen konvex ausgeführt. Die von ihm ausgebildete Auflagefläche 22 hat somit etwa die 20- bis 25-fache Größe der Querschnittsfläche des Schraubenschaftes 2.

Die Schraube 1 ist im Ausführungsbeispiel aus einem für die medizinische Verwendung geeigneten nicht-osseointegrierbaren Material, nämlich medizinischem Stahl, gefertigt. Der Schraubenschaft 2 weist zusätzlich zum Gewindebereich 8 einen gewindefreien, benachbart zum Schraubenkopf 6 angeordneten Schaftbereich 24 mit einem Schaftdurchmesser von etwa 1,2 mm auf.

Im ersten Gewindesegment 12 ist das Gewinde 16 im Ausführungsbeispiel als metrisches ISO-Gewinde, insbesondere als M-Gewinde, mit einem Außendurchmesser von etwa 1 mm ausgeführt. Im zweiten Gewindesegment 14, also dem Gewindesegment 14 zwischen dem ersten Gewindesegment 12 und dem Schraubenkopf 6, ist das Gewinde 18 hingegen als selbstschneidendes Gewinde 18 ausgeführt, und es weist hier einen Kerndurchmesser von etwa 1,2 mm auf.

Im Ausführungsbeispiel weist die Schraube eine Gesamtlänge von 11 mm auf; es können aber auch andere Gesamtlängen wie beispielsweise 8 oder 13 mm vorgesehen sein.

In FIG. 2 ist in Draufsicht eine Gewindemutter 26 gezeigt, die zur Verwendung mit der Schraube 1 vorgesehen ist. Die Gewindemutter 26 umfasst einen im Wesentlichen tellerartig ausgeführt Grundkörper 28, der in einem Zentralbereich mit einer ein von seiner Parameterwahl an das Gewinde 16 der Schraube 1 angepasstes Innengewinde 30 tragenden Gewindebohrung 32 versehen ist. Die Gewindemutter 26 kann somit auf einfache Weise auf das erste Gewindesegment 12 aufgeschraubt werden. Die Gewindemutter 26 weist einen an den Außendurchmesser des Schraubenkopfs 6 angepassten Außendurchmesser, im Ausführungsbeispiel somit ebenfalls 5 mm, auf.

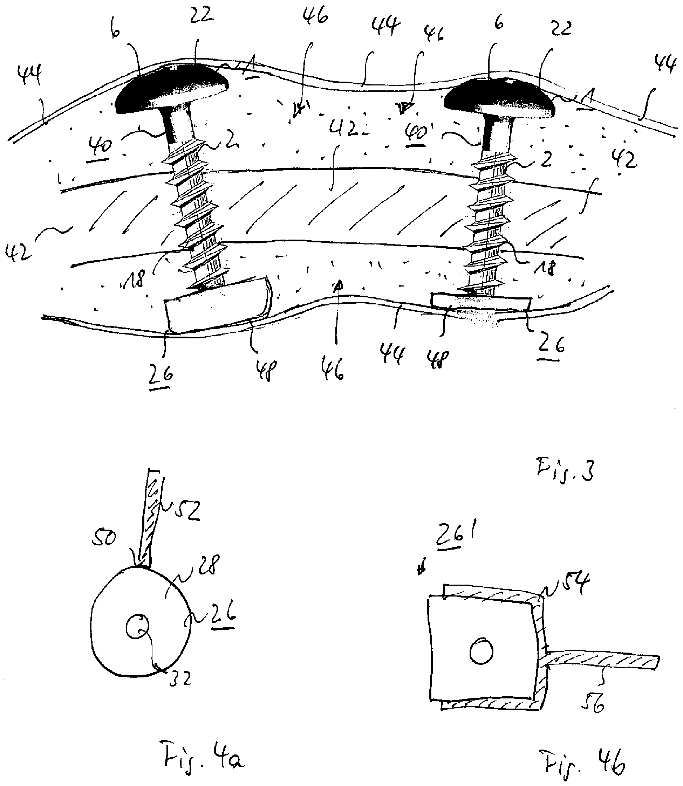

Die Kombination aus einer Schraube 1 mit einer aufgeschraubten Gewindemutter 26 bildet jeweils ein System 40 zur Modifikation eines menschlichen oder tierischen Knochens. Zwei von diesen Systemen 40 sind in FIG. 3 im inserierten, d. h. im Knochen verankerten, Zustand gezeigt.

Die jeweils aus einer Schraube 1 und einer zugeordneten Gewindemutter 26 gebildeten Systeme 40 sind für ein an sich als erfindungsgemäß angesehenes Wirkungsprinzip zur Erreichung einer angestrebten Knochenaugmentation oder eines Knochenaufbaus ausgelegt. Dabei wird die flächig ausgedehnte, im Wesentlichen konvex geformte Auflageflächge 22 des jeweiligen Schraubenkopfes 6 zwischen dem Knochen 42 und dem Periost oder der Knochenhaut 44 positioniert. Dies erfolgt, indem der jeweilige Schraubenschaft 2 mit seinem zweiten Gewindebereich 14, in dem das Gewinde 10 als selbstschneidendes Gewinde 18 ausgeführt ist, in den Knochen 42 eingedreht und die jeweilige Schraube 1 somit dort verankert wird. Die Schraube 1 wird dabei derart eingedreht, dass zwischen dem Schraubenkopf 6 und der Grenzfläche des Knochens 42 noch ein gewisser, vorgebbarer Restabstand verbleibt. Dadurch wird das Periost oder die Knochenhaut 44 bzw. ein jeweiliger Hautlappen, der/die von der jeweiligen Auflagefläche 22 abgestützt wird, vom eigentlichen Knochen 42 abgehoben, so dass ein Freiraum 46 zwischen Knochen 42 und Knochenhaut 44 entsteht. In diesen Freiraum 46 können aus den Gefäßwänden des expandierten Gewebes Perzyten einwandern, die nachfolgend eine Knochenneubildung im geschaffenen Freiraum 46 bewirken, oder es kann Knochen vom umliegenden Knochen aus in diesen Freiraum 46 einwachsen.

Durch die Ausführung und Formgestaltung des jeweiligen Schraubenkopfs 6, nämlich insbesondere vergleichsweise flach und ohne scharfe Kanten in der Auflagefläche 22 und im Wesentlichen konvex, ist formbedingt die Möglichkeit oder Wahrscheinlichkeit einer Verletzung oder Perforation des Periosts oder der Knochenhaut 44 weitgehend minimiert. Der Schraubenkopf 6 stützt das Periost zeltartig ab, wobei die eingetragenen Kräfte auf die vergleichsweise große Auflagefläche 22 harmonisch verteilt werden.

Durch die genannte Ausgestaltung der Schraube 1, insbesondere mit den mindestens zwei Gewindesegmenten 12, 14, kann diese auch in therapeutischen Situationen eingesetzt werden, in denen der zur Verfügung stehende Knochen 42 eine vergleichsweise geringe Substanz und Dicke aufweist. Dieser Aspekt ist in FIG. 3 ebenfalls gezeigt. Durch die in Kombination mit der jeweiligen Schraube 1 verwendete Gewindemutter 26 wird nämlich auch an dem dem Schraubenkopf 6 gegenüberliegenden Schaftende 20 eine Auflagefläche 48 für das dort befindliche Periost oder die dort befindliche Knochenhaut 44 geschaffen, so dass auch auf dieser Seite des Knochens 42 der zum Knochenaufbau erwünschte Freiraum 46 geschaffen werden kann.

Bei der Insertion des jeweiligen Systems 40 in den Knochen 42 ist vorgesehen, zunächst die Schraube 1 im Bereich ihres selbstschneidenden Gewindes 18 in den Knochen 42 einzudrehen und erst anschließend die zugeordnete Gewindemutter 26 auf das erste Gewindesegment 12 aufzuschrauben. Um dem Rechnung zu tragen, kann die Gewindemutter 26 mit einem Montage-Hilfsmittel versehen sein, dass dem Bediener oder Operateur bei der Einbringung die Positionierung, Justierung und/oder Endmontage, also das Aufschrauben, erleichtert. In FIG. 4 sind zwei Ausführungsbeispiele für eine solchermaßen vorbereitete Gewindemutter 26, 26' gezeigt; selbstverständlich ist auch eine beliebige Vielzahl weiterer analoger Ausführungsformen hierfür denkbar.

In FIG. 4a ist in Draufsicht ein Ausführungsbeispiel einer Gewindemutter 26 mit im wesentlichen tellerartig oder mit kreisförmiger Grundfläche ausgeformtem Grundkörper 28 gezeigt, an den über eine Sollbruchstelle 50 in der Art eines Griffs, eines Hebels oder eines Betätigungsnippels ein Montage-Hilfskörper 52 angeformt ist. Dieser kann nach dem Aufschrauben der Gewindemutter 26 auf das erste Gewindesegment 12 beispielsweise durch Abbrechen im Bereich der Sollbruchstelle 50 auf besonders einfache Weise entfernt werden.

Alternativ dazu zeigt FIG. 4b in Draufsicht eine Gewindemutter 26', deren Grundkörper 28, bezogen auf seine Längsachse, mit einer nicht runden, nämlich als Vierkant, ausgeführten Außenkontur ausgestaltet ist. **In** diesem Fall könnte die Gewindemutter 26' mit einem zugeordneten, beispielsweise mitgelieferten, Halte- oder Montagewerkzeug 54 versehen sein, an das ebenfalls ein Betätigungsgriff oder -hebel 56 angeformt ist, und das nach Einsetzen der Gewindemutter 26 abgezogen oder auf sonstige Weise entfernt werden kann. Das Montagewerkzeug 54 kann somit als wegwerfbares Einwegprodukt, alternativ aber auch als sterilisierbares, wiederverwendbares Werkzeug ausgeführt sein.

### Bezugszeichenliste

- 1: Schraube
- 2: Schraubenschaft
- 4: erstes Schaftende
- 6: Schraubenkopf
- 8: Gewindebereich
- 10: Gewinde
- 12,14: Gewindesegment
- 16,18: Gewinde
- 20: Schaftende
- 22: Auflagefläche
- 24: Schaftbereich
- 26,26': Gewindemutter
- 28: Grundkörper
- 30: Innengewinde
- 32: Gewindebohrung
- 40: System
- 42: Knochen
- 44: Knochenhaut
- 46: Freiraum
- 48: Auflagefläche
- 50: Sollbruchstelle
- 52: Montage-Hilfskörper
- 54: Montagewerkzeug
- 56: Betätigungsgriff

## Patentansprüche

1. Schraube (1) zur Verwendung bei der Modifikation eines menschlichen oder tierischen Knochens (42), insbesondere zum therapeutischen Einsatz im Bereich der plastischen oder dentalen Chirurgie, mit einem in einem Gewindebereich (8) mit einem Gewinde (10) versehenen Schraubenschaft (2), an dessen erstem Schaftende (4) ein im Wesentlichen konvex geformter Schraubenkopf (6) angeordnet ist, und dessen Gewindebereich (8) mindestens zwei Gewindesegmente (12, 14) umfasst, **dadurch gekennzeichnet, dass** in einem ersten, distalen, im Bereich des dem Schraubenkopf (6) gegenüberliegenden Endes (20) des Schraubenschafts (2) positionierten Gewindesegment (12) das Gewinde (16) als ISOmetrische Gewindeschraube mit einem Außendurchmesser von weniger als dem Außendurchmesser, vorzugsweise von weniger als dem Kerndurchmesser, des Gewindes (18) in einem zweiten, am Schraubenschaft (2) zwischen dem Schraubenkopf (6) und dem ersten Gewindesegment (12) angeordneten, als selbstschneidendes Gewinde (18) ausgestalteten Gewindesegment (14) ausgeführt ist.

2. Schraube (1) nach Anspruch 1, deren Schraubenschaft (2) zusätzlich zum Gewindebereich (8) einen gewindefreien, bevorzugt benachbart zum Schraubenkopf (6) angeordneten Schaftbereich (24) mit einem Schaftdurchmesser von mindestens 0,8 mm und höchstens 1,5 mm, bevorzugt von etwa 1,2 mm, aufweist.

3. Schraube (1) nach Anspruch 1 oder 2, deren Gewinde (18) im zweiten Gewindesegment (14) einen Kerndurchmesser von mindestens 0,8 mm und höchstens 1,5 mm, bevorzugt von etwa 1,2 mm, aufweist.

4. Schraube (1) nach einem der Ansprüche 1 bis 3, deren Gewinde (16) im ersten Gewindesegment (12) als M-Gewinde (16) mit einem Außendurchmesser von mindestens 0,8 mm und höchstens 1,5 mm, bevorzugt von etwa 1 mm, ausgeführt ist.

5. System (40) zur Modifikation eines menschlichen oder tierischen Knochens (2), mit einer Schraube (1) nach einem der Ansprüche 1 bis 4, und mit einer Gewindemutter (26, 26') , die einen im Wesentlichen tellerartig ausgeführt Grundkörper (28) aufweist, der in einem Zentralbereich mit einer ein von seiner Parameterwahl an das Gewinde (16) des ersten Gewindesegments (12) der Schraube (1) angepasstes Innengewinde (30) tragenden Gewindebohrung (32) versehen ist.

6. System (40) nach Anspruch 5, bei dem an den Grundkörper (28) der Gewindemutter (26, 26') über eine Sollbruchstelle (50) ein Montage-Hilfskörper (52) angeformt ist.

7. System (40) nach Anspruch 5 oder 6, bei dem der Grundkörper (28) der Gewindemutter (26, 26'), bezogen auf seine Längsachse, mit einer nicht runden, vorzugsweise als Mehrkant, besonders bevorzugt als Vier- oder Sechskant, ausgeführten Außenkontur ausgestaltet ist.

## Claims

1. A screw (1) for use in the modification of a human or animal bone (42), in particular for therapeutic use in the field of plastic or dental surgery, with a screw shaft (2) which is provided with a thread (10) in a threaded region (8) and which has a substantially convexly-shaped screw head (6) disposed at the first shaft end (4) thereof, and the threaded region (8) thereof comprises at least two threaded segments (12, 14), **characterized in that** in a first, distal threaded segment (12) which is positioned in the region of the end (20) of the screw shaft (2) opposite the screw head (6), the thread (16) is constructed as an ISO metric screw thread with an outer diameter of less than the outer diameter, preferably less than the core diameter, of the thread (18) in a second threaded segment (14) disposed on the screw shaft (2) between the screw head (6) and the first threaded segment (12) and formed as a self-tapping thread (18).

2. The screw (1) as claimed in claim 1, the screw shaft (2) of which, in addition to the threaded region (8), having a thread-free shaft region (24) which is preferably disposed adjacent to the screw head (6), with a shaft diameter of at least 0.8 mm and at most 1.5 mm, preferably of approximately 1.2 mm.

3. The screw (1) as claimed in claim 1 or claim 2, the thread (18) in the second thread segment (14) of which having a core diameter of at least 0.8 mm and at most 1.5 mm, preferably of approximately 1.2 mm.

4. The screw (1) as claimed in one of claims 1 to 3, the thread (16) in the first thread segment (12) of which being constructed as an M thread (16) with an outer diameter of at least 0.8 mm and at most 1.5 mm, preferably of approximately 1 mm.

5. A system (40) for the modification of a human or animal bone (2), with a screw (1) as claimed in one of claims 1 to 4, and with a threaded nut (26, 26') which has a substantially plate-like main body (28) which is provided in a central area with a threaded hole (32) carrying an internal thread (30) which is adapted to the thread (16) of the first threaded segment (12) of the screw (1) by selection of its parameters.

6. The system (40) as claimed in claim 5, in which an installation assistance member (52) is integrally formed on the main body (28) of the threaded nut (26, 26') via a predetermined breaking point (50).

7. The system (40) as claimed in claim 5 or claim 6 in which, with respect to its longitudinal axis, the main body (28) of the threaded nut (26, 26') is formed with a non-circular outer contour, preferably constructed as a polygon, particularly preferably as a square or hexagon.

## Revendications

1. Vis (1), destinée à une utilisation lors de la modification d'un os (42) humain ou animal, notamment à une utilisation thérapeutique dans le domaine de la chirurgie plastique ou dentaire, dotée d'une tige de vis (2) munie d'un filetage (10) dans une zone filetée (8), sur la première extrémité de tige (4) de laquelle est placée une tête de vis (6) de forme sensiblement convexe et dont la zone filetée (8) comprend au moins deux segments filetés (12, 14), **caractérisée en ce que** dans un premier segment fileté (12) distal, positionné dans la zone de l'extrémité (20) de la tige de vis (2) opposée à la tête de vis (6), le filetage (16) est réalisé sous la forme d'une vis filetée métrique, avec un diamètre extérieur inférieur au diamètre extérieur, de préférence inférieur au diamètre d'âme du filetage (18) dans un deuxième segment fileté (14), placé sur la tige de vis (2), entre la tête de vis (6) et le premier segment fileté (12), conçu sous la forme d'un filetage (18) autotaraudeur.

2. Vis (1) selon la revendication 1, dont la tige de vis (2) comporte, additionnellement à la zone filetée (8) une zone de tige (24) exempte de filetage, placée de préférence au voisinage de la tête de vis (6), avec un diamètre de tige d'au moins 0,8 mm et d'au plus 1,5 mm, de manière préférentielle, d'au moins 1,2 mm.

3. Vis (1) selon la revendication 1 ou 2, dont le filetage (18) présente dans le deuxième segment fileté (14) un diamètre d'âme d'au moins 0,8 mm et d'au plus 1,5 mm, de manière préférentielle, d'au moins 1,2 mm.

4. Vis (1) selon l'une quelconque des revendications 1 à 3, dont le filetage (16) dans le premier segment fileté (12) est réalisé sous la forme d'un filetage M (16), avec un diamètre extérieur d'au moins 0,8 mm et d'au plus 1,5 mm, de manière préférentielle, d'au moins 1 mm.

5. Système (40), destiné à la modification d'un os (2) humain ou animal, doté d'une vis (1) selon l'une quelconque des revendications 1 à 4 et doté d'un écrou fileté (26, 26'), qui comporte un corps de base (28) sensiblement réalisé sous la forme d'un plateau, qui dans une zone centrale, est muni d'un filetage taraudé (32) portant un taraudage (30) adapté par sa sélection de paramètre au filetage (16) du premier segment fileté (12) de la vis (1).

6. Système (40) selon la revendication 5, sur lequel sur le corps de base (28) de l'écrou fileté (26, 26'), un corps auxiliaire de montage (52) est surmoulé par l'intermédiaire d'un point de rupture théorique (50).

7. Système (40) selon la revendication 5 ou 6, sur lequel le corps de base (28) de l'écrou fileté (26, 26') est conçu avec un contour non rond, par rapport à zon axe longitudinal, de préférence réalisé sous la forme d'un polygone, de manière préférentielle d'un carré ou d'un hexagone.
